# EUROPEAN PATENT SPECIFICATION

(11) **EP 1 713 477 B1**
(45) Date of publication and mention of the grant of the patent: **01.10.2008**
(21) Application number: 05702261.8
(22) Date of filing: 13.01.2005
(51) Int. Cl.: A61K 31/4196, A61K 31/506, A61P 31/00

(54) **COMBINATION OF VORICONAZOLE AND AN ANTIFUNGAL CYP2C19 INHIBITOR**
KOMBINATION VON VORICONAZOL UND EINEM ANTIFUNGALEN CYP2C19-INHIBITOR
COMBINAISON DE VORICONAZOLE ET D'UN INHIBITEUR DE CYP2C19 ANTIFONGIQUE

(30) Priority: 04.02.2004 GB 0402491; 17.02.2004 US 545663 P
(43) Date of publication of application: 25.10.2006
(73) Proprietor: Pfizer Limited, Sandwich, Kent CT13 9NJ (GB); Pfizer Inc., New York NY 10017 (US)
(72) Inventor: HUMPHREY, Michael John, Sandwich, Kent CT13 9NJ (GB)
(74) Representative: Lane, Graham Mark Hamilton
(86) International application number: PCT/IB2005/000098
(87) International publication number: WO 2005/084671

(56) References cited:
- GHANNOUM M ET AL: "IN VITRO EVALUATION OF VORICONAZOLE IN COMBINATION WITH ANTIFUNGALS" INTERNATIONAL JOURNAL OF INFECTIOUS DISEASES, INTERNATIONAL SOCIETY FOR INFECTIOUS DISEASES, HAMILTON, CA, vol. 6, no. SUPPL 2, June 2002 (2002-06), pages 2S50-2S51, XP008058584 ISSN: 1201-9712 cited in the application

## Description

### Field of the Invention

This invention relates to a new combination therapy including voriconazole.

### Background to the Invention

(2R,3S)-2-(2,4-Difluorophenyl)-3-(5-fluoro-4-pyrimidinyl)-1-(1H-1,2,4-triazol-1-yl)-butan-2-ol, also known as voriconazole, is disclosed in EP-A-440372; see in particular Example 7. Voriconazole has the following structure: 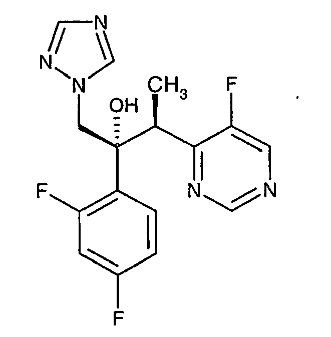 and is useful in the treatment of fungal infections.

The pharmacokinetics of voriconazole are characterised by saturable metabolism resulting in non-linear increases in exposure with increasing dose levels. Futhermore, drug exposure varies to a significant extent between subjects. Voriconazole is metabolised by the cytochrome P450 isozymes CYP2C19, CYP2C9 and CYP3A4. The major circulating metabolite, the structure of which is given below, results from N-oxidation.

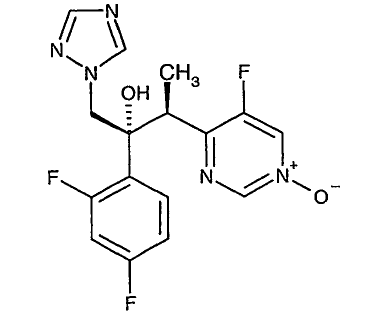

We have found that the metabolism of voriconazole is dependent to a large extent on the genotype of subjects being treated. One genotype metabolises voriconazole extensively, leading to rapid clearance of voriconazole from the body and, consequently, low plasma levels of voriconazole (ranging from about 0.6 to about 1.4 µg/ml). In this specification this genotype will be referred to as "extensive metabolisers". A second genotype can be characterised as a poor metaboliser of voriconazole: in this genotype, voriconazole is cleared much more slowly and therefore remains at higher levels in the body (ranging from about 3.5 to about 5.5 µg/ml). In this specification this genotype will be referred to as "poor metabolisers". The genotyping is believed to be due to a recessive gene: homozygous extensive metabolisers make up about 73% of the Caucasian and 35% of the Japanese population; heterozygous extensive metabolisers make up about 25% of the Caucasian and 46% of the Japanese population. By contrast, poor metabolisers make up only about 2% of the Caucasian and about 19% of the Japanese population.

It is now understood that the variability between genotypes with respect to the metabolism of voriconazole is dependent on the extent to which the enzyme cytochrome P450 2C19 (hereinafter referred to as CYP2C19) is present in the body: the enzyme CYP2C19 is present in extensive metabolisers, whereas poor metabolisers lack the functional enzyme. See, for example, M de Morais, G Wilkinson, J Blaisdell et al. J Biol Chem (1994), 269,15419-15422.

In practice, voriconazole is administered to both extensive and poor metabolisers without dose adjustment. However, the need for voriconazole to be present in sufficient quantities in plasma to exert a therapeutic effect on extensive metabolisers requires a high dose of the drug: the usual recommended daily dose is 400mg (200mg twice a day).
This dose in poor metabolisers results in higher systemic exposure which may lead to undesirable side effects. Moreover, the rapid-clearance of the drug by extensive metabolisers requires the compound to be administered twice a day to enable it to maintain plasma levels throughout the day and exert a therapeutic effect. The need for twice-daily therapy raises compliance issues if voriconazole is to be self-administered by the patient.

M. Ghannoum, N. Isham, M. Hossain and D. Sheehan, Int. J. Infect. Dis. (2002), Vol. 6 Supp. 2, 2S50 discloses in vitro combinations of voriconazole with antifungal agents including amphotericin B, Abelce^{™}, 5-fluorocytosine and fluconazole, and investigates their mechanistic synergy against a range of organisms. Combinations of voriconazole with fluconazole are stated to be 39% additive and 61 % indifferent.

M. Ghannoum, N. Isham and D. Sheehan, Abstracts of the Interscience Conference on Antimicrobial Agents and Chemotherapy (2002) 42, 385 also discloses in vitro combinations of voriconazole with amphotericin B, Abelcet^{™}, fluconazole, micafungin, ravuconazole and caspofungin. Combinations of voriconazole with fluconazole are stated to be 100% indifferent, ie no synergy of mechanism was observed.

H.J. Scherpbier, M.I. Hilhorst and T.W. Kuijpers, Clin. Infect. Dis. (2003) 37, 828, discloses the treatment of an AIDS patient with a combination of antiretroviral drugs and report an interaction between protease inhibitors and voriconazole when the latter was added to a patient's therapy to treat oesophageal candidiasis. The interaction involved liver function impairment and elevated plasma concentrations of lopinavir, nevirapine and amprenavir. Plasma concentrations of voriconazole were not measured in the patients.

N. Wood, K. Tan, L. Purkins, G. Layton, J. Hamlin, D. Kleinermans and D. Nichols, Br. J. Clin. Pharmacol. (2003) 56, 56 describes a study to determine the effects of the proton pump inhibitor omeprazole, a CYP2C19 inhibitor, on the steady state pharmacokinetics of voriconazole. The study concluded that omeprazole had no clinically relevant effect on voriconazole exposure, suggesting that no voriconazole dosage adjustment is necessary for patients in whom omeprazole therapy is initiated.

It would be desirable to devise a voriconazole therapy which eliminates or reduces intersubject variability. Furthermore, it would be desirable to devise a voriconazole therapy in which the administered dose of voriconazole was reduced. In addition, it would be desirable to devise a voriconazole therapy in which clearance of the drug from plasma was reduced, thereby allowing voriconazole to be administered once daily.

We have surprisingly found that inhibition of the CYP2C19 enzyme by co-administration of voriconazole with a second, different antifungal capable of inhibition of the activity of CYP2C19 markedly reduces metabolism in extensive metabolisers of voriconazole, causing the pharmacokinetic profile of such subjects to approximate to that of poor metabolisers. This results in markedly reduced intersubject variability and in therapeutic plasma levels being achieved at much lower doses of voriconazole. Furthermore, it results in reduced clearance of voriconazole from the body, enabling voriconazole to be present in sufficient plasma concentrations throughout the day with the potential to achieve a therapeutic effect when administered once a day.

### Summary of the Invention

The invention provides in a first aspect a therapeutic combination comprising voriconazole and fluconazole in specific quantities or weight ratios, defined in more detail hereinafter.

The invention also provides in a second aspect a pharmaceutical composition comprising a therapeutically effective amount of voriconazole and fluconazole, together with a pharmaceutically acceptable carrier or diluent.

The invention also provides in a third aspect a unit dosage form comprising a therapeutically effective amount of voriconazole and a therapeutically effective amount of fluconazole.

The invention further provides in a fourth aspect a kit comprising a plurality of separate containers, wherein at least one container contains voriconazole and at least one different container contains fluconazole.

The invention additionally provides in a fifth aspect the use of the above combination, composition, kit or unit dosage form in the manufacture of a medicament for the treatment of a fungal infection in a mammal.

Hereinafter the therapeutic combination, pharmaceutical composition, unit dosage form, kit, use and method of the present invention will be referred to jointly as "the combination of the present invention".

### Brief Description of Drawings

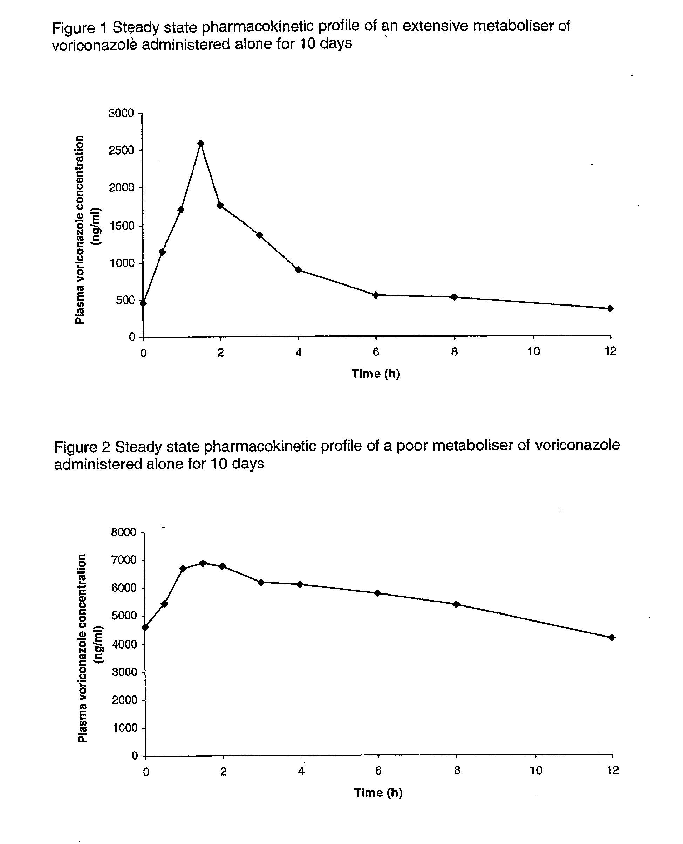

Fig. 1 Illustrates the pharmacokinetic profile of an extensive metaboliser of voriconazole when administered alone.
Fig. 2 illustrates the pharmacokinetic profile of a poor metaboliser of voriconazole when administered alone.
Fig. 3 Illustrates the effect of fluconazole on N-oxide metabolite formation in vitro in human liver microsome HL-MIX 101 (control pool).
Fig. 4 illustrates the effect of fluconazole on N-oxide metabolite formation in vitro in human liver microsome HH-92 (CYP2C19 poor metaboliser).
Fig. 5 illustrates the effect of fluconazole on N-oxide metabolite formation in vitro in human liver microsome HH-112 (CYP2C19 poor metaboliser).
Fig. 6 illustrates the effect of fluconazole on N-oxide metabolite formation in vitro in human liver microsome HH-100 (CYP2C19 extensive metaboliser).
Fig. 7 illustrates the effect of fluconazole on N-oxide metabolite formation in rCYP2C19.
Fig. 8 illustrates the effect of fluconazole on N-oxide metabolite formation in rCYP3A4.
Figs. 9 and 10 are pharmacokinetic profiles of two poor metabolisers of voriconazole when administered alone and together with fluconazole.
Figs. 11 and 12 are pharmacokinetic profiles of two extensive metabolisers of voriconazole when administered alone and together with fluconazole.

### Detailed Description of Preferred Embodiments

One part of the combination of the present invention is voriconazole. Voriconazole is disclosed in EP-A-440372 ; see in particular Example 7. As described in more-detail below, voriconazole may be administered as the free base, or in the form of a salt, solvate or prodrug thereof.

The other part of the combination of the present invention is the antifungal CYP2C19 inhibitor fluconazole, which has a Ki of 2µM- see L.C. Winkers, C.J. Wurden, E. Storch et al, Drug Metab Dispos (1996) 24, 610-4.

It is preferred that the antifungal CYP2C19 inhibitor is capable of selectively inhibiting the metabolism of voriconazole by the enzyme CYP2C19 over the enzyme CYP3A4. Selectivity for the isozymes can be measured by relative inhibitory potency against (S)-mephenytoin 4-hydroxylase and testosterone 6 beta hydroxylase activity: measurement of the latter activity Is described in Funa and Imaoka (1987) Biochem. Blophys. Acta. 926, 349-358. More specifically the selectivity can be demonstrated by the effects on voriconazole N- oxidation using 25 and 2500µM substrate concentrations to assess the effects on CYP2C19 (high affinity enzyme) and 3A4 (low affinity enzyme) respectively. The methodology for measuring voriconazole N-oxidation by cytochrome P450 isozymes is described by Hyland, Jones and Smith (2003) Drug Metabolism and Disposition, 31 (5), 540-547. The precise level of selectivity required depends on the antifungal CYP2C19 inhibitor and its pharmacokinetics and variability between subjects: however, we prefer that the antifungal CYP2C19 inhibitor exhibits a selectivity for. CYP2C19 over CYP3A4 (as measured by their relative IC₅₀ values in the aforementioned Hyland et al paper) of 2 to 10, and preferably 3 to 6. Without wishing to be bound by theory, it is believed that selective inhibition of CYP2C19 over CYP3A4 allows intersubject variability to be reduced and therapeutic plasma levels to be achieved at lower doses of voriconazole, while diminishing possible undesirable side-effects in the patient population under the current recommended dosage regimen. At low concentrations CYP2C19 is the predominant route of clearance while at higher concentrations the CYP3A4 route of metabolism becomes the major, non saturable, route of voriconazole clearance.

Preferably, the antifungal CYP2C19 inhibitor has a pharmacokinetic half-life suitable to permit the voriconazole / antifungal CYP2C19 inhibitor combination to maintain plasma levels throughout the day and achieve a therapeutic effect when administered once a day. Suitable half-lives range from 6 to 72 hours, preferably from 12 to 48 hours, and more preferably from 18 to 36 hours.

It is preferred that the antifungal CYP2C19 inhibitor is excreted from the body mainly as unchanged drug. Without wishing to be bound by theory, it is believed that an antifungal CYP2C19 inhibitor which is excreted from the body mainly as unchanged drug allows intersubject variability to be reduced both of the antifungal CYP2C19 inhibitor and voriconazole. Preferably from 50 to 99%, more preferably from 70 to 80% and most preferably about 75% of the drug is excreted from the body unchanged.

The antifungal CYP2C19 inhibitor exhibits antifungal activity. This confers the advantage that the combination exhibits no effect other than an antifungal effect. The second antifungal would preferably have an additive effect to antifungal activity and may preferably be approved for safe use in the same patient population. The overall antifungal effect depends, of course, on the specific infection being treated, the dose of voriconazole and antifungal CYP2C19 inhibitor administered, and the age, sex, weight and condition of the patient being treated.

The combination of the present invention comprises voriconazole and an antifungal CYP2C19 inhibitor. Voriconazole and the antifungal CYP2C19 inhibitor may be administered as the free acid or base, or in the form of a pharmaceutically acceptable salt, or solvate.

Pharmaceutically acceptable salts of voriconazole and the antifungal CYP2C19 inhibitor include the acid addition and base salts thereof.

Suitable acid addition salts are formed from acids which form non-toxic salts. Examples include the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

For a review on suitable salts, see "Handbook of Pharmaceutical Salts: Properties, Selection, and Use" by Stahl and Wermuth (Wiley-VCH, Weinheim, Germany, 2002).

A pharmaceutically acceptable salt of voriconazole or an antifungal CYP2C19 inhibitor may be readily prepared by mixing together solutions of voriconazole or the antifungal CYP2C19 inhibitor and the desired acid or base, as appropriate. The salt may precipitate from solution and be collected by filtration or may be recovered by evaporation of the solvent. The degree of ionisation in the salt may vary from completely ionised to almost non-ionised.

Voriconazole and the antifungal CYP2C19 inhibitor may exist in both unsolvated and solvated forms. The term 'solvate' is used herein to describe a molecular complex comprising voriconazole and/or the antifungal CYP2C19 inhibitor and one or more pharmaceutically acceptable solvent molecules, for example, ethanol. The term 'hydrate' is employed when said solvent is water.

Hereinafter all references to voriconazole and/or the antifungal CYP2C19 inhibitor include references to salts, solvates and complexes thereof and to solvates and complexes of salts thereof.

The combination of the invention includes voriconazole and an antifungal CYP2C19 inhibitor as hereinbefore defined, or polymorphs thereof.

As stated, the invention includes all polymorphs of voriconazole and/or the antifungal CYP2C19 inhibitor as hereinbefore defined.

Voriconazole contains an alcohol functionality (-OH), therefore some examples of prodrugs in accordance with the invention may include an ester or ether thereof, for example, by replacement of the hydrogen by phosphorylation to provide (2R,3S)-2-(2,4-difluorophenyl)-3-(5-fluoro-4-pyrimidinyl)-1-(1H-1,2,4-triazol-1-yl)-2-butyl dihydrogen phosphate as described in WO 97/28169 ; see in particular Example 3.

Similarly, the antifungal CYP2C19 inhibitor fluconazole also contains an alcohol functionality (-OH), therefore some examples of prodrugs in accordance with the invention may include an ester or ether thereof, for example, by replacement of the hydrogen by phosphorylation to provide 2-(2,4-difluorophenyl) 1,3-bis(1 H-1,2,4-triazol-1-yl)-2-propyl dihydrogen phosphate as described in WO 97/28169; see in particular. Example 1, or a pharmaceutically acceptable salt thereof, especially the disodium salt (Prodif^{®}). Further examples of replacement groups in accordance with the foregoing examples and examples of other prodrug types may be found in the aforementioned references.

The antifungal CYP2C19 inhibitor may contain one or more asymmetric carbon atoms and may therefore exist as two or more stereoisomers. Where the compound contains, for example, an aromatic moiety, tautomeric isomerism ('tautomerism') can occur. It follows that a single compound may exhibit more than one type of isomerism.

Included within the scope of the present invention are all stereoisomers, geometric isomers and tautomeric forms of the antifungal CYP2C19 inhibitor, including compounds exhibiting more than one type of isomerism, and mixtures of one or more thereof. Also included are acid addition or base salts wherein the counterion is optically active, for example, D-lactate or L-lysine, or racemic, for example, DL-tartrate or DL-arginine.

*Cis*/*trans* isomers may be separated by conventional techniques well known to those skilled in the art, for example, chromatography and fractional crystallisation.

Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC).

Alternatively, the racemate (or a racemic precursor) may be reacted with a suitable optically active compound, for example, an alcohol, or, in the case where the antifungal CYP2C19 inhibitor contains an acidic or basic moiety, an acid or base such as tartaric acid or 1-phenylethylamine. The resulting diastereomeric mixture may be separated by chromatography and/or fractional crystallization and one or both of the diastereoisomers converted to the corresponding pure enantiomer(s) by means well known to a skilled person.

Chiral compounds used in the combination of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% isopropanol, typically from 2 to 20%, and from 0 to 5% of an alkylamine, typically 0.1 % diethylamine. Concentration of the eluate affords the enriched mixture.

Stereoisomeric conglomerates may be separated by conventional techniques known to those skilled in the art - see, for example, "Stereochemistry of Organic Compounds" by E L Eliel (Wiley, New York, 1994).

The combination of the invention comprises an amount of antifungal CYP2C19 inhibitor effective as an antifungal and effective to inhibit the action of the CYP2C19 enzyme, preferably to selectively inhibit said enzyme over the CYP3A4 enzyme. The precise dose administered depends on various factors such as the age, sex, weight and condition of the patient being treated. However, we prefer that the administered dose is from 5 to 500 mg, more preferably 10 to 250 mg, even more preferably 50 mg to 200 mg, and most preferably about 75 to about 125 mg.

The combination of the invention comprises an amount of voriconazole effective to act as an antifungal. The precise dose administered depends on various factors such as the age, sex, weight and condition of the patient being treated. However, we prefer that the administered dose is from about 5 to about 600 mg, preferably about 10 to about 500 mg, more preferably about 20 to about 300 mg, and most preferably about 25 to about 250 mg. A dose of about 200 mg is particularly preferred: this confers a significant advantage according to the present invention in that the dose of voriconazole can be halved from its usual 400 mg levels, thus minimising undesirable side effects.

In preferred embodiments, the combination of the present invention is to be administered to a patient once a day. This confers the particular advantage of better patient compliance. However, administration two, three or four times a day is also envisaged as being within the scope of the present invention, although the dose per administration would be reduced further.

The weight ratio in which the components of the combination of the invention are administered varies depending on various factors such as the age, sex, weight and condition of the patient being treated. However, we prefer that voriconazole and the antifungal CYP2C19 inhibitor are administered in a weight ratio ranging from about 1:4 to 6:1, preferably 1:2 to 3:1, and more preferably 3:2 to 5:2.

Fungal infections which may be treated by the combination of the invention have been extensively described in the literature, including EP-A-440372, and include topical infections, mucosal infections, such as vaginal candidiasis, oesophageal and oropharyngeal candidiasis, and systemic infections. Furthermore, the combination of the invention.may be used to treat allergic reactions, such as allergic rhinosinusitis. Fungal infections which may be treated by the combination of the invention include those caused by, *inter alia*, *Candida spp, Trichophyton spp, Microsporum spp, Epidermophyton floccosum, Cryptococcus neoformans, Aspergillus spp, Fusarium spp, Scedosporium spp, Coccidioides immitis, Paracoccidioides brasiliensis, Histoplasma spp, Blastomyces dermatiditis, Alternaria spp, Exophiala spp, Fonsecaea pedrosoi, Penicillium marneffei, Phialophora spp* or *Paecilomyces lilacinus.* It will be appreciated that reference to treatment is intended to include prophylaxis as well as the alleviation of established symptoms.

In the combination of the present invention, voriconazole and the antifungal CYP2C19 inhibitor may be administered, in terms of dosage forms, either separately or in conjunction with each other; and in terms of their time of administration, either simultaneously or sequentially. Thus, the administration of voriconazole may be prior to, concurrent with, or subsequent to the administration of the antifungal CYP2C19 inhibitor. The time in between administration may vary within a 24-hour dosing interval.

The unit dosage form of the invention is a dosage form in which both voriconazole and the antifungal CYP2C19 inhibitor are present. It may be a solid formulation for oral administration such as a tablet, a capsule containing a particulate, liquid, or powder, a lozenge (including liquid-filled), a chew, a gel, a solid solution, a liposome, a film (including muco-adhesive), an ovules, a spray or a liquid formulation, a parenteral formulations (typically an aqueous solution which may contain excipients as defined hereinbelow), or a formulation for topical administration to the skin or mucosa, (ie dermally or transdermally) such as a hydrogel, a lotion, a solution, a cream, an ointment, a dusting powder, a dressing, a foam, a skin patch, a wafer, an implant, a sponge, a fibre, a bandage or a microemulsion. Preferably the unit dosage form of the invention is a tablet or capsule, especially a tablet, containing voriconazole and the antifungal CYP2C19 inhibitor.

Compounds of the combination of the invention intended for pharmaceutical use may be administered as crystalline or amorphous products. They may be obtained, for example, as solid plugs, powders, or films by methods such as precipitation, crystallization, freeze drying, spray drying, or evaporative drying. Microwave or radio frequency drying may be used for this purpose.

Generally, the composition of the invention will be administered as a formulation in association with one or more pharmaceutically acceptable excipients. The term "excipient" is used herein to describe any ingredient other than the compounds of the invention. The choice of excipient will to a large extent depend on factors such as the particular mode of administration, the effect of the excipient on solubility and stability, and the nature of the dosage form.

Pharmaceutical compositions suitable for the delivery of compounds of the present invention and methods for their preparation will be readily apparent to those skilled in the art. Such compositions and methods for their preparation may be found, for example, in 'Remington's Pharmaceutical Sciences', 19the Edition (Mack Publishing Company, 1995).

The compounds of the invention may be administered orally. Oral administration may involve swallowing, so that the compounds enter the gastrointestinal tract, or buccal or sublingual administration may be employed by which the compounds enter the blood stream directly from the mouth.

Formulations suitable for oral administration include solid formulations such as tablets, capsules containing particulates, liquids, or powders, lozenges (including liquid-filled), chews, multi- and nano-particulates, gels, solid solution, liposome, films (including muco-adhesive), ovules, sprays and liquid formulations.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, 11 (6), 981-986 by Liang and Chen (2001).

For tablet dosage forms, depending on dose, the drug may make up from 1 wt% to 80 wt% of the dosage form, more typically from 5 wt% to 60 wt% of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from 1 wt% to 25 wt%, preferably from 5 wt% to 20 wt% of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally include surface active agents, such as sodium lauryl sulfate and polysorbate 80, and glidants such as silicon dioxide and talc. When present, surface active agents may comprise from 0.2 wt% to 5 wt% of the tablet, and glidants may comprise from 0.2 wt% to 1 wt% of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 wt% to 10 wt%, preferably from 0.5 wt% to 3 wt% of the tablet.

Other possible ingredients include antioxidants, colourants, flavouring agents, preservatives and taste-masking agents.

Exemplary tablets contain up to about 80% drug, from about 10 wt% to about 90 wt% binder, from about 0 wt% to about 85 wt% diluent, from about 2 wt% to about 10 wt% disintegrant, and from about 0.25 wt% to about 10 wt% lubricant.

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

The formulation of tablets is discussed in "Pharmaceutical Dosage Forms: Tablets, Vol. 1", by H. Lieberman and L. Lachman, Marcel Dekker, N.Y., N.Y., 1980 (ISBN 0-8247-6918-X).

Solid formulations for oral administration may be formulated to be immediate and/or modified release. Modified release formulations include delayed-, sustained-, pulsed-, controlled-, targeted and programmed release.

Suitable modified release formulations for the purposes of the invention are described in US Patent No. 6,106,864. Details of other suitable release technologies such as high energy dispersions and osmotic and coated particles are to be found in Verma et al, Pharmaceutical Technology On-line, 25(2), 1-14 (2001). The use of chewing gum to achieve controlled release is described in WO 00/35298.

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques. An example of a needle free injection is Powderject^{™}.

Parenteral formulations are typically aqueous solutions which may contain excipients such as salts, carbohydrates and buffering agents (preferably, to a pH of from 3 to 9), but, for some applications, they may be more suitably formulated as a sterile nonaqueous solution or as a powdered, dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of voriconazole and the antifungal CYP2C19 inhibitor used in the preparation of parenteral solutions may be increased by the use of appropriate formulation techniques, such as the incorporation of solubility-enhancing agents.
Formulations for use with needle-free injection administration comprise a compound of the invention in powdered form in conjunction with a suitable vehicle such as sterile, pyrogen-free water. For example, the aqueous solubility of voriconazole may be increased by formulating it with one or more poloxamers as described in UK Patent Application No. 0327390.1 Alternatively, the aqueous solubility of voriconazole may be increased by formulating it with a sulfobutyl ether cyclodextrin such as those disclosed in WO 91/11172 and WO 94/02518. A formulation of voriconazole with a sulfobutyl ether cyclodextrin is described in WO 98/58677.

Formulations for parenteral administration may be formulated to be immediate and/or modified/controlled release. Controlled/modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLA microspheres.

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958 by Finnin and Morgan (October 1999). Topical administration may also be achieved using a patch, such as a transdermal iontophoretic patch.

Other means of topical administration include delivery by electroporation, iontophoresis, phonophoresis, sonophoresis and microneedle or needle-free (*e.g*. Powderject^{™}, Bioject^{™}, *etc*.) injection.

Formulations for topical administration may be formulated to be immediate and/or modified/controlled release. Controlled/modified release formulations include delayed-, sustained-, pulsed-, controlled-, targeted and programmed release.
It is within the scope of the present invention that compositions containing voriconazole and the antifungal CYP2C19 inhibitor may conveniently be combined in the form of a kit suitable for coadministration of the compositions.

Thus the kit of the invention comprises two or more separate pharmaceutical compositions, at least one of which contains voriconazole and another contains the antifungal CYP2C19 inhibitor, and means for separately retaining said compositions, such as a container, divided bottle, or divided foil packet. An example of such a kit is the familiar blister pack used for the packaging of tablets, capsules and the like.

The kit of the invention is particularly suitable for administering different dosage forms, for example, oral and parenteral, for administering the separate compositions at different dosage intervals, or for titrating the separate compositions against one another. To assist compliance, the kit typically comprises directions for administration and may be provided with a so-called memory aid.

### Examples

### Example 1

### Inhibition of Cytochrome P450 activity in human liver microsomes by fluconazole

The data in Table 1 below were determined according to the method described in Meier et al. (1985) Anal.Biochem, 151,286-291, and in Funa and Imaoka (1987) Biochem. Biophys. Acta. 926, 349-358.

**TABLE 1**

| **Cytochrome P450** | **IC₅₀ (µM)** |
|---|---|
| CYP2C19 (S-Mephenytoin 4-hydroxylase) | 47 |
| CYP3A4 (Testosterone 6 beta -hydroxylase) | 214 |

As demonstrated by the data above, fluconazole exhibits selective inhibition of the enzyme CYP2C19 over CYP3A4.

### Example 2

### In vitro metabolism of voriconazole in human liver microsomes and rCYP2C19 and rCYP3A4: Selectivity of enzyme inhibition by fluconazole

The following incubation mix (final concentrations) was used for all assays described in this study; 50mM potassium phosphate buffer (pH 7.4), 5mM MgCl₂, 5mM isocitric acid, 1 U/mL isocitric acid dehydrogenase. Reducing equivalents required for P450 metabolism were provided by NADPH, which was regenerated using isocitric acid/isocitric acid dehydrogenase.

### Time Course Studies

Incubations were performed in the following human liver microsome preparations; control batch HL-MIX-101 (prepared from a pool of 60 donors); two donors genotyped as CYP2C19 poor metabolisers (HH-92, HH-112) and a CYP2C19 extensive metaboliser with high CYP2C19 activity (HH-100). Relative CYP2C19 and CYP3A4 activities are shown in Table 2 below:

**TABLE 2**

| Human liver microsome characteristics | | | | | |
|---|---|---|---|---|---|
| | CYP2C19 genotype | Protein (mg/mL) | Total P450 (pmol/mL) | CYP3A4 activity (nmol/min/mg protein) | CYP2C19 activity (nmol/min/mg protein) |
| HL-MIX-101 | Mixed pool | 20.4 | 16 | 5.2 | 0.050 |
| HH-92 | PM | 17.6 | 14 | 8.2 | 0.004 |
| HH-112 | PM | 20.9 | 9 | 1.6 | 0.0012 |
| HH-100 | EM | 18.7 | 7 | 3.8 | 0.18 |

| | | | | | |
|---|---|---|---|---|---|
| (PM = poor metaboliser; EM = extensive metaboliser) | | | | | |

Additional incubations were performed with microsomes prepared from insect cells transfected with recombinant CYP2C19 and CYP3A4 (BDGENTEST^{®} supersomes and Panvera baculosomes, respectively)

A series of preliminary studies were required to ensure the formation of the N-oxide metabolite was linear during the incubation period using the aforementioned reaction mixture. Here human liver microsomes (1 mg/mL) were pre-incubated in the presence of the substrate voriconazole (25µM final concentration) prior to the addition of the NADPH. Note for studies using rCYP2C19 and rCYP3A4, pre-incubations were performed with NADPH and the reaction initiated by the addition of the substrate voriconazole. Aliquots (1 mL) of the reaction mixture were collected over time (0-60mins) and added to 4mL of dichloromethane containing 50µL of internal standard (2-(2,4-difluorophenyl)-3-(4-pyrimidyl)-1-(1H-1,2,4-triazol-1-yl)-2-butanol; 15µg/mL). Samples were rotary mixed for 10 minutes, and then centrifuged at 3000 rpm for 5min (4°C). The upper aqueous layers were removed and discarded. The lower organic layer was evaporated to dryness at 37°C under a stream of N₂. Samples were subsequently reconstituted in 100µL mobile phase A (see below) and 25µL injected onto the HPLC.

Samples were analysed on an Agilent 1100 Series UV-HPLC. Chromatographic separation was performed using a Hichrom 100-5C18 column (150 x 4.6 mm) at a flow rate of 1 mU min with UV-λ set at 254nm. The mobile phases used were; (A) 0.1 M ammonium phosphate in 70:30 H₂O:MeOH, (pH adjusted to 7.0, prior to the addition of MeOH) and (B) MeOH, using the following gradient; 0-2min (0% B), 2-20 (0-100% B), 20-23 (100% B), 23.1 (0% B). Column was allowed to re-equilibrate for 7 mins prior to next injection. Typical retention times were 7.9min for fluconazole; 11.7min for the N-oxide metabolite; 13.1 min for the internal standard and 14.5min for voriconazole.

The inhibitory potential of fluconazole against voriconazole N-oxidation was investigated in human liver microsome and rCYP2C19 and rCYP3A4 preparations. The final concentrations of fluconazole studied were 0 0.1, 1, 10, 100 and 1000µM.

Human liver microsomes were incubated at 1 mg/mL for 60mins, rCYP2C19 at 10pmol CYP/mL for 60min and rCYP3A4 at 100pmol CYP/mL for 20mins. For each matrix, aliquots (4mL) of the reaction mixture were pre-warmed at 37°C in a waterbath followed by the addition of 200µl NADPH (20mM) and 40µL of respective fluconazole solutions. (0-100mM). Reactions were initiated by the addition of 50µL voriconazole (2mM). At the end of the incubation time aliquots (n=3) were taken from each incubation mix and added into tubes containing 4mL of dichloromethane and 50µl of internal standard (2-(2,4-difluorophenyl)-3-(4-pyrimidyl)-1-(1H-1,2,4-triazol-1-yl)-2-butanol; 15µg/mL). The extraction and analysis procedures followed that described above.

At the concentrations reported, formation of the N-oxide metabolite was linear to 60mins in human liver microsomes preparations and rCYP2C19. In rCYP3A4 linearity was measured to 20mins.

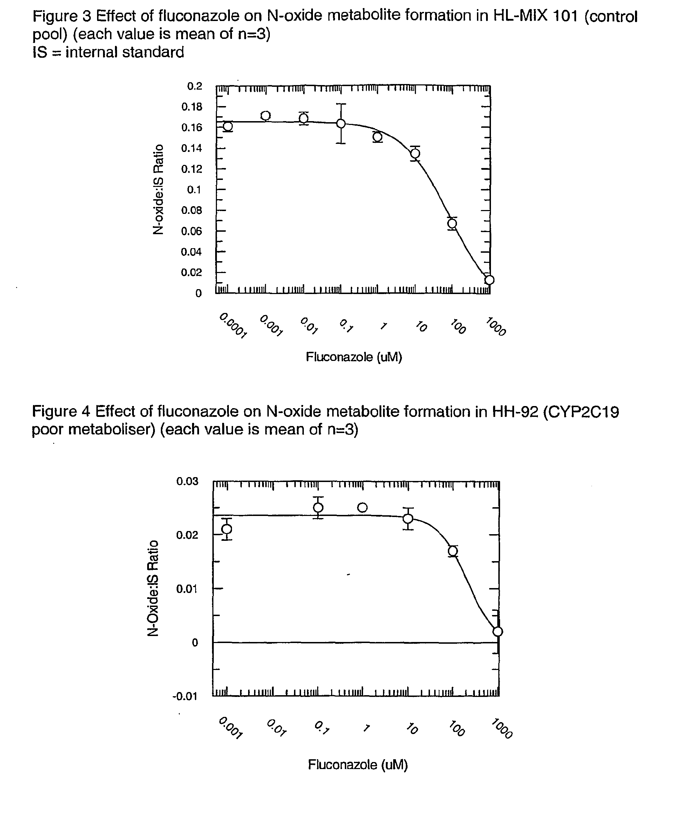

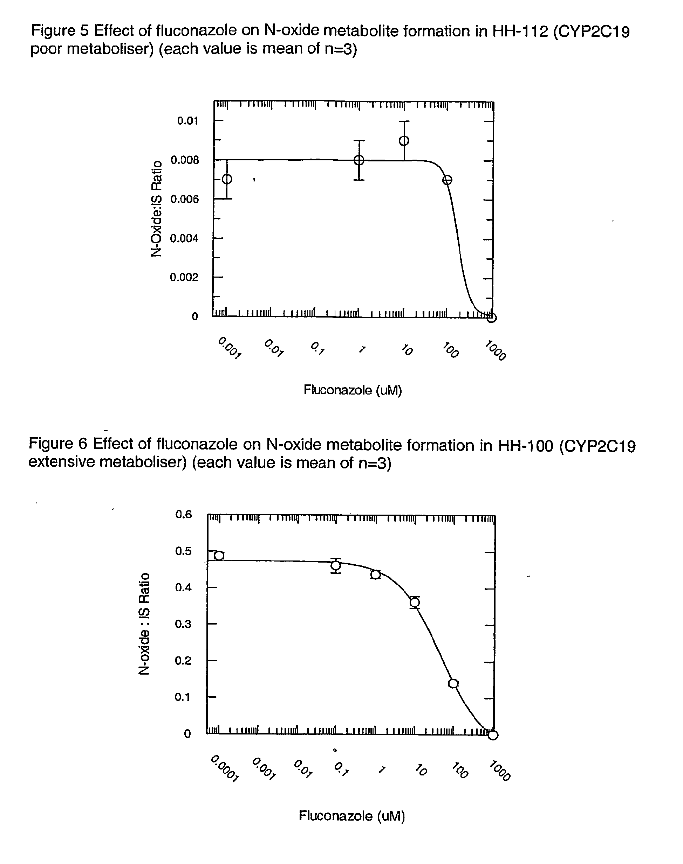

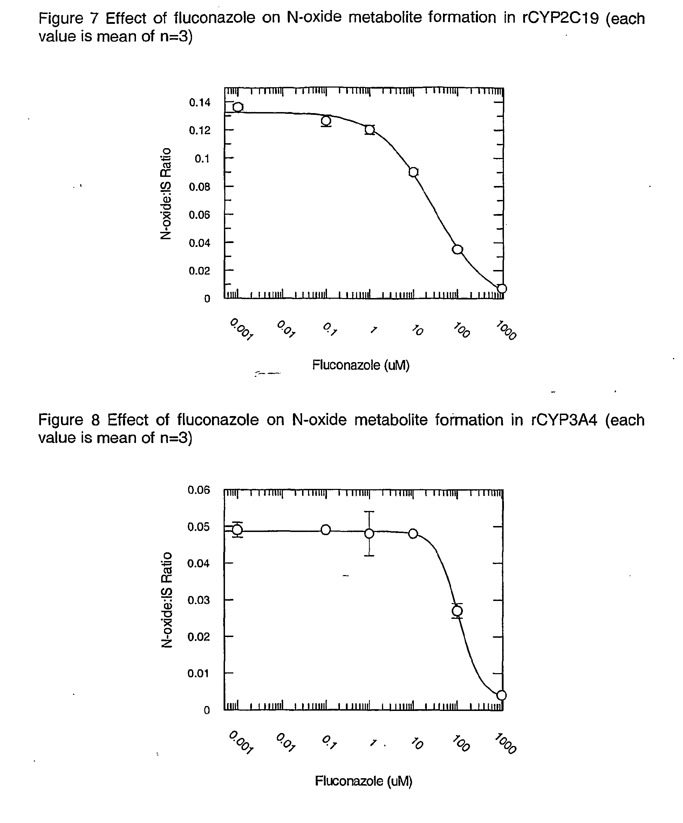

The inhibitory potential (IC₅₀) of fluconazole against metabolism of voriconazole to its N-oxide metabolite was studied in a pool of human liver microsomes (HL-MIX-101) as well as individual donors genotyped as being poor (HH-92 and HH-112) or extensive CYP2C19 metabolisers (HH-100). Furthermore the CYP2C19 PM's were subdivided as having either high and low CYP3A4 metabolic capacities. To better define the selectivity of fluconazole for inhibition of CYP3A4- or CYP2C19-mediated metabolism of voriconazole, rCYP3A4 or rCYP2C19 were also investigated. Results are summarised in Figures 3-8 and collated in Table 3.

**TABLE 3**

| IC₅₀ determination for fluconazole in human liver microsomes (CYP2C19 PM & EM'S) and rCYP2C19 and rCYP3A4 | |
|---|---|
| | IC₅₀(µM) |
| HL-MIX-101 (mixed pool) | 82 |
| HH-92 (poor metaboliser) | 214 |
| HH-112 (poor metaboliser) | 175 |
| HH-100 (extensive metaboliser) | 50 |
| rCYP2C19 | 29 |
| rCYP3A4 | 106 |

In CYP2C19 poor metaboliser human liver microsomes fluconazole is a weak inhibitor of voriconazole N-oxidation (IC₅₀~175µM and 214µM). More potent inhibitions were observed in the CYP2C19 extensive metaboliser microsomes (IC₅₀~50µM). Experiments using rCYP2C19 and rCYP3A4 demonstrate fluconazole to be a more potent inhibitor of CYP2C19 mediated N-oxidation (IC₅₀~29µM) compared to CYP3A4 (IC₅₀~106µM).

The overall finding from these experiments highlights a 3-4 fold selectivity for fluconazole towards inhibition of CYP2C19 mediated voriconazole N-oxidation over CYP3A4 mediated N-oxidation.

### Example 3

### Clinical trials

A study was conducted to investigate the effect of co-administered fluconazole on steady state pharmacokinetics of voriconazole in healthy male subjects and to assess the safety and toleration of co-administered fluconazole and voriconazole. Ten healthy male subjects aged 21-55 were recruited to ensure at least 8 subjects completed the study, including two CYP2C19 poor metabolisers. The two treatments were:
1. a 400mg twice daily oral loading dose of voriconazole on day 1, followed by 200mg twice daily oral doses on Days 2-3 and a single 200mg oral dose on the morning of Day 4.
2. a 400mg twice daily oral loading dose of voriconazole on Day 1, followed by 200mg twice daily oral doses on Days 2-3 and a single 200mg oral dose on the morning of Day 4, plus a single oral dose of 400mg fluconazole with the morning dose of voriconazole on Day 1, and single oral doses of 200mg fluconazole once daily on the morning of Days 2-5.

Subjects were randomly assigned to receive treatments either in the sequence of voriconazole followed by voriconazole plus fluconazole, or in the sequence voriconazole plus fluconazole followed by voriconazole.

Blood samples were taken on Days 4, 5 and 6 of each treatment period to provide plasma concentrations of voriconazole for calculation of the pharmacokinetic parameters Cₘₐₓ, Tₘₐₓ, AUC_{T}, AUCₜ and AUC for Day 4 of each treatment period. For the subjects classed as CYP2C19 extensive metabolisers, these parameters were then used to compare voriconazole alone versus voriconazole plus fluconazole. The same parameters for the subjects classified as CYP2C19 poor metabolisers were also used for comparison with the data from the extensive metabolisers.

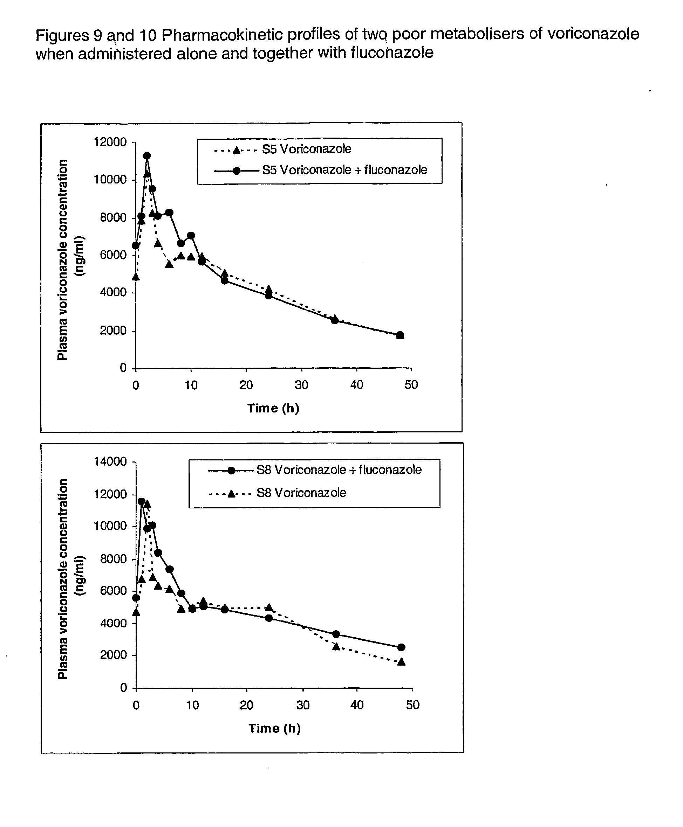

The results are shown in Figures 9 and 10 (poor metabolisers) and 11 and 12 (extensive metabolisers). Figures 9 and 10 clearly show that fluconazole has little or no effect on the pharmacokinetics of voriconazole in poor metabolisers.

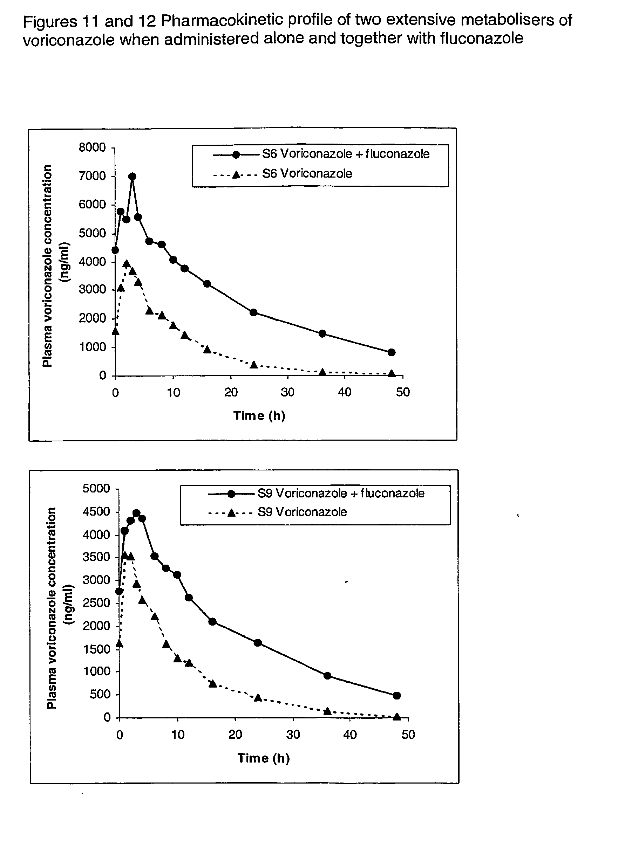

However, Figures 11 and 12 show the effect of 200mg fluconazole once daily on the standard dose of voriconazole of 200mg twice daily in an extensive metaboliser. It should be noted that at 24 hours after the last dose the voriconazole levels are maintained at >2000ng/ml which is considered to be in excess of the plasma concentration required for efficacy. Furthermore, the half-life of voriconazole in extensive metabolisers is increased to >18 hours.

This shows that co-administration of voriconazole with fluconazole results in reduced clearance of voriconazole from the body of an extensive metaboliser. This enables voriconazole to be present in sufficient plasma concentrations throughout the day so that it can achieve a therapeutic effect in an extensive metaboliser when administered once a day.

Furthermore, the data indicates that by co-administration of voriconazole with fluconazole, plasma levels of voriconazole may be achieved at much reduced doses of voriconazole - a reduction by a factor of two for once daily dosing and four for twice daily dosing can be envisaged. In addition, the data indicates that significantly reduced variability in the patient population can be achieved.

The data shows that therapeutic plasma levels of voriconazole may be achieved at much reduced doses of voriconazole. This may allow poor metabolisers or heterozygous extensive metabolisers to reduce the administered voriconazole doses and blood levels by a factor of 2 to 4. The lower systemic exposure achieved may in turn minimise any undesirable side effects.

## Claims

1. A therapeutic combination comprising voriconazole and an antifungal CYP2C19 inhibitor, wherein the voriconazole is present in an amount of 5 to 600 mg and the antifungal CYP2C19 inhibitor is present in an amount of 5 to 500 mg and wherein the antifungal CYP2C19 inhibitor is fluconazole.

2. A combination according to claim 1, wherein the voriconazole is present in an amount of 20 to 300 mg and the antifungal CYP2C19 inhibitor is present in an amount of 50 to 200 mg.

3. A combination according to claim 1, wherein the voriconazole is present in an amount of 25 to 250 mg and the antifungal CYP2C19 inhibitor is present in an amount of 75 to 125 mg.

4. A therapeutic combination comprising voriconazole and an antifungal CYP2C19 inhibitor, wherein the voriconazole and antifungal CYP2C19 inhibitor are present in a weight ratio of 1:4 to 6:1 and wherein the antifungal CYP2C19 inhibitor is fluconazole.

5. A combination according to claim 4, wherein the voriconazole and antifungal CYP2C19 inhibitor are present in a weight ratio of 1:2 to 3:1.

6. A combination according to claim 4, wherein the voriconazole and antifungal CYP2C19 inhibitor are present in a weight ratio of 3:2 to 5:2.

7. A pharmaceutical composition comprising a therapeutically effective amount of voriconazole and an antifungal CYP2C19 inhibitor, together with a pharmaceutically acceptable carrier or diluent, wherein the antifungal CYP2C19 inhibitor is fluconazole and the voriconazole and fluconazole are present in the amounts given in any one of claims 1 to 3 or the ratios given in any one of claims 4 to 6.

8. A kit comprising a plurality of separate containers, wherein at least one container contains voriconazole and at least one different container contains an antifungal CYP2C19 inhibitor, wherein the antifungal CYP2C19 inhibitor is fluconazole and the voriconazole and fluconazole are present in the amounts given in any one of claims 1 to 3 or the ratios given in any one of claims 4 to 6.

9. A unit dosage form comprising a therapeutically effective amount of voriconazole and a therapeutically effective amount of an antifungal CYP2C19 inhibitor, wherein the antifungal CYP2C19 inhibitor is fluconazole and the voriconazole and fluconazole are present in the amounts given in any one of claims 1 to 3 or the ratios given in any one of claims 4 to 6.

10. The use of a combination according to any one of claims 1 to 6, a composition according to claim 7, a kit according to claim 8 or a unit dosage form according to claim 9 in the manufacture of a medicament for the treatment of a fungal infection in a mammal.

## Patentansprüche

1. Therapeutische Kombination, umfassend Voriconazol und einen antimykotischen CYP2C19-Inhibitor, wobei das Voriconazol in einer Menge von 5 bis 600 mg vorhanden ist und der antimykotische CYP2C19-Inhibitor in einer Menge von 5 bis 500 mg vorhanden ist und wobei der antimykotische CYP2C19-Inhibitor Fluconazol ist.

2. Kombination gemäß Anspruch 1, wobei das Voriconazol in einer Menge von 20 bis 300 mg vorhanden ist und der antimykotische CYP2C19-Inhibitor in einer Menge von 50 bis 200 mg vorhanden ist.

3. Kombination gemäß Anspruch 1, wobei das Voriconazol in einer Menge von 25 bis 250 mg vorhanden ist und der antimykotische CYP2C19-Inhibitor in einer Menge von 75 bis 125 mg vorhanden ist.

4. Therapeutische Kombination, umfassend Voriconazol und einen antimykotischen CYP2C19-Inhibitor, wobei das Voriconazol und der antimykotische CYP2C19-Inhibitor in einem Gewichtsverhältnis von 1:4 bis 6:1 vorhanden sind und wobei der antimykotische CYP2C19-Inhibitor Fluconazol ist.

5. Kombination gemäß Anspruch 4, wobei das Voriconazol und der antimykotische CYP2C19-Inhibitor in einem Gewichtsverhältnis von 1:2 bis 3:1 vorhanden sind.

6. Kombination gemäß Anspruch 4, wobei das Voriconazol und der antimykotische CYP2C19-Inhibitor in einem Gewichtsverhältnis von 3:2 bis 5:2 vorhanden sind.

7. Pharmazeutische Zusammensetzung, umfassend eine therapeutisch wirksame Menge von Voriconazol und einem antimykotischen CYP2C19-Inhibitor, zusammen mit einem pharmazeutisch verträglichen Träger oder Verdünnungsmittel, wobei der antimykotische CYP2C19-Inhibitor Fluconazol ist und das Voriconazol und das Fluconazol in Mengen vorhanden sind, die in einem der Ansprüche 1 bis 3 angegeben sind, oder in den Verhältnissen, die in einem der Ansprüche 4 bis 6 angegeben sind.

8. Kit, umfassend eine Vielzahl getrennter Behälter, wobei mindestens ein Behälter Voriconazol enthält und mindestens ein anderer Behälter einen antimykotischen CYP2C19-Inhibitor enthält, wobei der antimykotische CYP2C19-Inhibitor Fluconazol ist und das Voriconazol und das Fluconazol in Mengen vorhanden sind, die in einem der Ansprüche 1 bis 3 angegeben sind, oder in den Verhältnissen, die in einem der Ansprüche 4 bis 6 angegeben sind.

9. Einheitsdosierungsform, umfassend eine therapeutisch wirksame Menge von Voriconazol und eine therapeutisch wirksame Menge eines antimykotischen CYP2C19-Inhibitors, wobei der antimykotische CYP2C19-Inhibitor Fluconazol ist und das Voriconazol und das Fluconazol in Mengen vorhanden sind, die in einem der Ansprüche 1 bis 3 angegeben sind, oder in den Verhältnissen, die in einem der Ansprüche 4 bis 6 angegeben sind.

10. Verwendung einer Kombination gemäß einem der Ansprüche 1 bis 6, einer Zusammensetzung gemäß Anspruch 7, einem Kit gemäß Anspruch 8 oder einer Einheitsdosierungsform gemäß Anspruch 9 bei der Herstellung eines Medikaments zur Behandlung einer mykotischen Infektion bei einem Säuger.

## Revendications

1. Combinaison thérapeutique comprenant du voriconazole et un inhibiteur de CYP2C19 antifongique, dans laquelle le voriconazole est présent en une quantité allant de 5 à 600 mg et l'inhibiteur de CYP2C19 antifongique est présent en une quantité allant de 5 à 500 mg, l'inhibiteur de CYP2C19 antifongique étant le fluconazole.

2. Combinaison selon la revendication 1, dans laquelle le voriconazole est présent en une quantité allant de 20 à 300 mg et l'inhibiteur de CYP2C19 antifongique est présent en une quantité allant de 50 à 200 mg.

3. Combinaison selon la revendication 1, dans laquelle le voriconazole est présent en une quantité allant de 25 à 250 mg et l'inhibiteur de CYP2C19 antifongique est présent en une quantité allant de 75 à 125 mg.

4. Combinaison thérapeutique comprenant du voriconazole et un inhibiteur de CYP2C19 antifongique, le voriconazole et l'inhibiteur de CYP2C19 antifongique étant présents selon un rapport pondéral compris entre 1:4 et 6:1, et l'inhibiteur de CYP2C19 antifongique étant le fluconazole.

5. Combinaison selon la revendication 4, dans laquelle le voriconazole et l'inhibiteur de CYP2C19 antifongique sont présents selon un rapport pondéral compris entre 1:2 et 3:1.

6. Combinaison selon la revendication 4, dans laquelle le voriconazole et l'inhibiteur de CYP2C19 antifongique sont présents selon un rapport pondéral compris entre 3:2 et 5:2.

7. Composition pharmaceutique comprenant une quantité thérapeutiquement efficace de voriconazole et d'un inhibiteur de CYP2C19 antifongique, avec un support ou un diluant pharmaceutiquement acceptable, l'inhibiteur de CYP2C19 antifongique étant le fluconazole, et le voriconazole et le fluconazole étant présents dans les quantités indiquées selon l'une quelconque des revendications 1 à 3 ou dans les rapports indiqués selon l'une quelconque des revendications 4 à 6.

8. Kit comprenant une pluralité de récipients séparés, dans lequel l'un au moins des récipients contient du voriconazole et au moins un récipient différent contient un inhibiteur de CYP2C19 antifongique, l'inhibiteur de CYP2C19 antifongique étant le fluconazole, et le voriconazole et le fluconazole étant présents dans les quantités indiquées selon l'une quelconque des revendications 1 à 3 ou dans les rapports indiqués selon l'une quelconque des revendications 4 à 6.

9. Forme posologique unitaire comprenant une quantité thérapeutiquement efficace de voriconazole et une quantité thérapeutiquement efficace d'un inhibiteur de CYP2C19 antifongique, l'inhibiteur de CYP2C19 antifongique étant le fluconazole, et le voriconazole et le fluconazole étant présents dans les quantités indiquées selon l'une quelconque des revendications 1 à 3 ou dans les rapports indiqués selon l'une quelconque des revendications 4 à 6.

10. Utilisation d'une combinaison selon l'une quelconque des revendications 1 à 6, d'une composition selon la revendication 7, d'un kit selon la revendication 8 ou d'une forme posologique unitaire selon la revendication 9 dans la fabrication d'un médicament pour le traitement d'une infection fongique chez un mammifère.
